(19) Europäisches Patentamt
European Patent Office
Office européen des brevets

(11) Veröffentlichungsnummer : **0 369 563 B1**

(12) # EUROPÄISCHE PATENTSCHRIFT

(45) Veröffentlichungstag der Patentschrift : **14.04.93 Patentblatt 93/15**

(51) Int. Cl.[5] : **C07C 405/00**

(21) Anmeldenummer : **89250091.9**

(22) Anmeldetag : **17.11.89**

(54) **Verfahren zur Herstellung von Carbacyclin-Derivaten durch stereoselektive Einführung der 5,6-Doppelbindung.**

Die Akte enthält technische Angaben, die nach dem Eingang der Anmeldung eingereicht wurden und die nicht in dieser Patentschrift enthalten sind.

(30) Priorität : **17.11.88 DE 3839155**

(43) Veröffentlichungstag der Anmeldung : **23.05.90 Patentblatt 90/21**

(45) Bekanntmachung des Hinweises auf die Patenterteilung : **14.04.93 Patentblatt 93/15**

(84) Benannte Vertragsstaaten : **AT BE CH DE ES FR GB GR IT LI LU NL SE**

(56) Entgegenhaltungen :
**EP-A- 0 038 131**
**GB-A- 2 129 427**

(56) Entgegenhaltungen :
**TETRAHEDRON LETTERS, Band 22, Nr. 42, 1981, Seiten 4185-4188, Pergamon Press Ltd, Oxford, GB; B.E. MARYANOFF et al.: "Trans stereoselectivity in the reaction of (4-carboxybutylidene)triphenylphosphorane with aromatic aldehydes"**
**SYNTHESIS, Nr. 4, April 1988, Seiten 310-313, Stuttgart, DE; N. MONGELLI et al.: "1-Fluorocycloalkanecarboxylic acids in the synthesis of 16-Achiral 16-fluoro-9a-carbaprostacyclin derivatives"**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Harre, Michael**
**Fröaufstrasse 8**
**W-1000 Berlin 41 (DE)**
Erfinder : **Westermann, Jürgen**
**Elberfelder Strasse 19**
**W-1000 Berlin 21 (DE)**

EP 0 369 563 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Carbacyclin-Derivaten durch stereoselektive Einführung der 5,6-Doppelbindung, wobei der gewünschte E-Isomerenanteil im erhaltenen E/Z-Isomerengemisch deutlich erhöht werden kann.

Bei der Synthese von Carbacyclinen, wie z.B. Iloprost, stellt die stereoselektive Einführung der α-Kette einen wichtigen Syntheseschritt dar.

In der Literatur wird bisher nur eine Möglichkeit beschrieben, diese 5,6-trisubstituierte Doppelbindung stereoselektiv durch Hydrierung eines 1,3-Diens in Gegenwart eines Aryltricarbonylchrom-Komplexes in die gewünschte E-Form zu überführen [J. of Organic Chemistry 49, 4096 (1984) und 53, 1227 (1988)]. Der Nachteil dieses Verfahrens ist jedoch eine zu geringe Ausbeute, hervorgerufen durch unvollständige Umsetzung und eine zu große Zahl an Synthesestufen. Außerdem ist für Carbacycline mit einer Dreifachbindung in der unteren Kette dieses Verfahrens nicht geeignet, da eine partielle Hydrierung der Dreifachbindung erfolgt [s. J. of Organic Chemistry 49, 4096 (1984)].

In J. of Organic Chemistry 44, 2880 (1979) wird noch über eine weitere nicht selektive Methode zur Einführung der α-Kette mit Sulfoximinen berichtet.

K.C. Nicolaou et al. (J.C.S. Chem. Commun. 1978, 1067) erhalten ein Gemisch aus 5(E)- und 5(Z)-Carbacyclin durch eine nicht selektive Wittig-Reaktion mit einem 11,15-ungeschützten Keton.

Gleicherweise wird in Synthesis 1988, 310 die Einführung der α-Kette in ein 15-Fluorcyclohexyl-Carbacyclin mit einem angeblichen 70/30 E/Z Verhältnis durch ein Wittig-Reaktion beschrieben.

Es sind zwar Verfahren und Methoden zur Überführung von unerwünschten Z-Isomeren in gewünschte E-Isomeren (z.B. DOS 33 38 932) sowie Spaltungsreaktionen des Z-Isomeren mit Osmiumtetroxid zum wiederverwertbaren Ausgangsketon (H. Vorbrüggen et al., Synthesis 1985,925) bekannt.

Diese weiteren Isomerisierungs- bzw. Spaltungsreaktionen zur Erhöhung des E-Anteils erfordern einen beträchtlichen zusätzlichen synthetischen Aufwand. Daher kommt einem Verfahren zur Verbesserung des E/Z-Verhältnisses eine große Bedeutung zu.

Es wurde nun gefunden, daß die 11,15-Tetrahydropyranyl-geschützten OH-Gruppen (wie in DOS 28 45 770 beschrieben) in den Ausgangsketonen für die Wittig-Reaktion auch ungeschützt eingesetzt werden können. Das E/Z-Verhältnis in den dargestellten Carbacyclin-Derivaten erhöht sich dann sogar überraschend von 60:40 auf 85:15.

Dies bedeutet den Wegfall weiterer Synthesestufen bei der Herstellung der biologisch aktiven Carbacyclin-Derivate und damit eine zusätzliche Ausbeutesteigerung an Carbacyclinen gegenüber dem bisher bekannten Verfahren. Den Reaktionsparametern kommt hierbei eine besondere Bedeutung zu, wie anschließend gezeigt werden wird.

Die Erfindung betrifft ein Verfahren zur Herstellung von Carbacyclinen der Formel I

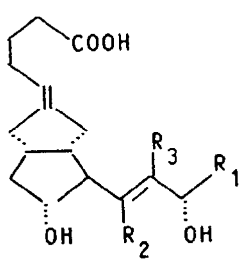

(I),

worin

$R_1$ eine geradkettige oder verzweigte $C_2$-$C_7$-Alkinyl-Gruppe und

$R_2$, $R_3$ Wasserstoff bedeuten können, wobei die 13,14-Doppelbindung trans-Konfiguration besitzt, das dadurch gekennzeichnet ist, daß man ein bicyclisches Keton der Formel II

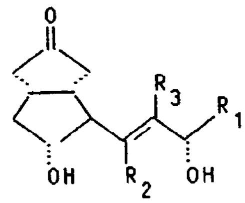

(II),

worin $R_1$, $R_2$ und $R_3$ die oben angegebenen Bedeutungen haben, mit 5-10 Equivalenten eines Reagenz der Formel VI,

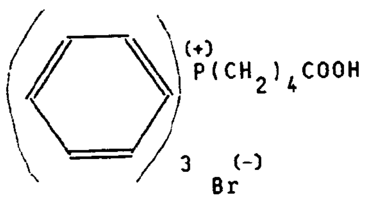

(VI),

in Gegenwart von 8-12 Equivalenten einer Base, bei 0-40°C in 0,5 bis 4 Std. in den Lösungsmitteln Dimethoxyethan, 1,3-Dimethylimidazolidin-2-on, Dimethylformamid, Dimethylsulfoxid, Tetrahydrofuran, Toluol, Dibenzo-18-krone-6 oder in deren Gemischen umsetzt.

Bei der Reaktion von II mit VI wird bevorzugt die Base Kalium-tert.-butylat eingesetzt. Als weitere Basen kommen aber auch Lithium- und Natrium-tert.-butylat in Betracht. Als Basen eignen sich ferner Metallhydride wie z.B. Lithiumhydrid, Natriumhydrid oder Kaliumhydrid, Metallamide wie z.B. Natriumamid, Kaliumamid, Natrium-bis-trimethylsilylamid oder Lithium-bis-trimethylsilylamid, dialkylsubstituierte Metallamide wie Natrium-, Lithium- oder Magnesiumdiisopropylamid sowie alle organischen oder anorganischen Basen mit ausreichender Basizität in aprotischen Lösungsmitteln.

Als geradkettige oder verzweigte $C_2$-$C_7$ Alkinylgruppen kommen in Betracht:
$-C{\equiv}CH$, $-CH_2-C{\equiv}CH$, $-CH_2-C{\equiv}C-C_3H_7$, $-CH_2-CH_2-C{\equiv}C-CH_3$,

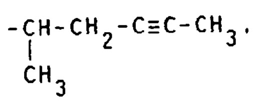

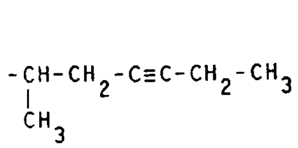

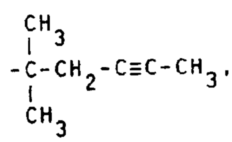

usw.

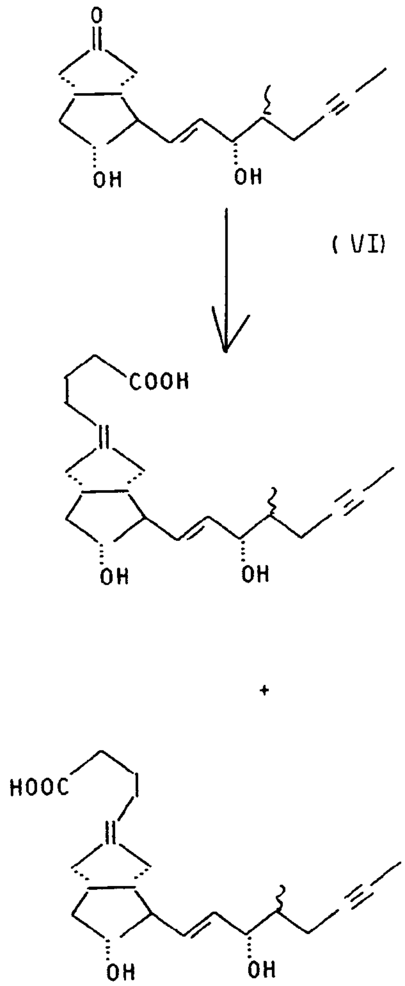


(III)

(IV)

+

(V)

Der Einfluß des Lösungsmittels auf das E/Z-Verhältnis bei Umsetzung des Ketons III mit dem Reagenz VI wird am Beispiel von Iloprost (IV) in der nachfolgenden Tabelle I deutlich.

Tabelle 1

| Lsgm. | eq Keton (III) | eq Wittig-Reag. (VI) | eq KOtBu | T (°C) | t (h) | Verhältnis (%E / %Z) (IV : V) |
|---|---|---|---|---|---|---|
| DMSO | 1.0 | 6 | 12 | 22 | 2 | 75/25 |
| DMSO/Tol 2:1 | 1.0 | 6 | 12 | 22 | 2 | 70/30 |
| DMSO/Tol 1:1 | 1.0 | 6 | 12 | 22 | 2 | 71/29 |
| DMSO/Tol 1:3 | 1.0 | 6 | 12 | 22 | 2 | 72/28 |
| DMSO/Tol 1:9 | 1.0 | 6 | 12 | 22 | 2 | 75/25 |
| Tol/18-K-6 | 1.0 | 6 | 12 | 22 | 2 | 75/25 |
| Toluol | 1.0 | 6 | 12 | 22 | 2 | 75/25 |
| DMF | 1.0 | 6 | 12 | 22 | 1 | 78/22 |
| DMI | 1.0 | 6 | 12 | 22 | 2 | 69/31 |
| Ether | 1.0 | 6 | 12 | 22 | 2 | 76/24 |
| Dioxan | 1.0 | 6 | 12 | 22 | 2 | 76/24 |
| THF | 1.0 | 6 | 12 | 22 | 2 | 78/22 |
| DME | 1.0 | 2 | 4 | 22 | 4 | 73/27 |
| DME | 1.0 | 4 | 8 | 22 | 2 | 78/22 |
| DME | 1.0 | 6 | 12 | 22 | 2 | 82/18 |

Tabelle 2 zeigt den Einfluß der Reaktionstemperatur auf das E/Z-Verhältnis bei der Umsetzung von III zu IV und V.

**Tabelle 2**

| Lsgm. | eq Keton (III) | eq Wittig-Reag. (VI) | eq KOtBu | T (°C) | t (h) | Verhältnis (%E / %Z) (IV : V) |
|---|---|---|---|---|---|---|
| DME | 1.0 | 6 | 12 | 0 | 3 | 85/15 |
| DME | 1.0 | 6 | 12 | 10 | 2 | 81/19 |
| DME | 1.0 | 6 | 12 | 30 | 1.5 | 78/22 |
| DME | 1.0 | 6 | 12 | 40 | 1.5 | 76/24 |
| DME | 1.0 | 6 | 12 | 50 | 0.5 | 74/26 |
| DME | 1.0 | 6 | 12 | 60 | 0.5 | 73/24 |

Tabelle 3 zeigt den Einfluß der Stöchiometrie des Wittigreagenzes (VI) auf das E/Z-Verhältnis bei Umsetzung von III zu IV und V:

Tabelle 3

| Lsgm. | eq Keton (III) | eq Wittig-Reag.(VI) | eq KOtBu | T (°C) | t (h) | Verhältnis (%E / %Z) (IV : V) |
|---|---|---|---|---|---|---|
| DMSO | 1.0 | 6 | 12 | 22 | 2 | 75/25 |
| DMSO | 1.0 | 3 | 6 | 22 | 16 | 72/28 |
| DME | 1.0 | 2 | 4 | 22 | 2 | 73/24 |
| DME | 1.0 | 3 | 6 | 22 | 2 | 77/23 |
| DME | 1.0 | 4 | 8 | 22 | 2 | 78/22 |
| DME | 1.0 | 5 | 10 | 22 | 2 | 80/20 |
| DME | 1.0 | 6 | 12 | 22 | 2 | 82/18 |
| DME | 1.0 | 6 | 12 | 0 | 4 | 85/15 |
| DME | 1.0 | 10 | 20 | 0 | 0.5 | 85/15 |

Wie aus den Tabellen 1-3 ersichtlich, werden bei Verwendung von 5-6 eq Wittig-Reagenz VI und 10-12 eq an Base gute E/Z-Werte erhalten.
Werden höhere molare Verhältnisse genommen (→ 10 eq), so werden ebenfalls hohe E/Z-Werte erhalten, aus ökonomischen Gründen ist ein allzuhoher Überschuß nicht anzustreben. Niedrigere molare Verhältnisse ergeben ein ungünstigeres E/Z-Verhältnis.

In den Tabellen 1, 2 und 3 und im nachfolgenden Text haben die Abkürzungen folgende Bedeutungen:

DME Dimethoxyethan
DMI 1,3-Dimethylimidazolidin-2-on
DMF Dimethylformamid
DMSO Dimethylsulfoxid
THF Tetrahydrofuran
Tol Toluol
18-K-6 Dibenzo-18-krone-6

Das Ausgangsmaterial der Formel III ist in EP 268548 beschrieben. Verbindungen der allgemeinen Formel II mit anderen Substituenten für $R_1$, $R_2$ und $R_3$ lassen sich analog darstellen.

**BEISPIEL**

5-[[(E)-(1S,5S,6R,7R)-7-Hydroxy-6-[(E)-(3S,4RS)-3-hydroxy-4-methyl-1-octen-6-inyl]-bicyclo[3.3.0]-oct-3-yliden]]pentansäure (IV)

Unter Stickstoffatmosphäre werden 63,71 g (0,144 mol) trockenes Carboxybutyltriphenylphosphoniumbromid in 180 ml abs. Dimethoxyethan vorgelegt und auf 0°C abgekühlt. Bei 0°C werden innerhalb von 15 min. 32,25 g (0,228 mol) Kaliumtert.-butylat so zugegeben, daß die Innentemperatur 10°C nicht übersteigt. Hierbei tritt eine Orangefärbung auf. Unter Rühren wird über einen Zeitraum von 45 min auf 0°C abgekühlt. Anschließend werden 6,62 g (23,9 mmol) (E)-(1S,5S,-6R,7R)-7-Hydroxy-6-[(E)(3S,4RS)-3-hydroxy-4-methyl-1-octen-6-inyl]- bicyclo-[3.3.0]-octan-3-on (III), gelöst in 20 ml abs. Dimethoxyethan, innerhalb von 5 min bei 0°C zugetropft und bei dieser Temperatur gerührt. Nach 2 h wird die Reaktionslösung auf 480 ml Eiswasser gegeben und 1 h gerührt (pH 13-14). Die Reaktionslösung wird mit 32,5 g Citronensäure (65 ml einer 50%igen wäßrigen Lösung) auf pH 3-4 angesäuert. Die Lösung wird 3 x mit je 120 ml Essigester extrahiert, die vereinten Essigesterphasen 2 x mit je 300 ml halbgesättigter Natriumchloridlösung gewaschen und die Essigesterphase über

Natriumsulfat getrocknet, filtriert und der Rückstand mit 50 ml Essigester nachgewaschen. Nach Abdampfen des Essigesters wird der Rückstand mit 240 ml Essigester ausgerührt. der helle Feststoff abfiltriert und der Filterrückstand mit 100 ml Essigester nachgewaschen. Die Essigesterlösung wird am Rotationsverdampfer eingeengt. (Rohprodukt: 16,82 g, enthält nach HPLC die Isomeren (IV) und (V) im Verhältnis 82:18). Das so vorgereinigte Produkt (enthält noch Triphenylphosphinoxid und Wittigsalze) wird mit MPLC an Kieselgel (0.04-0.063 mm) chromatographiert. Die Säule wurde mit Kieselgel und 1 .3 l Hexan/Essigester/Methanol/Essigsäure 70:30:1.5:0.5 naß aufgezogen und mit Hexan/Essigester/Methanol chromatographiert, wobei das Verhältnis Essigester zu Methanol konstant bei 95:5 gehalten wurde und der Hexananteil kontinuierlich reduziert wurde. Zuerst wurde das unpolarere Z-Isomer (V) (1,5 g) erhalten. Eine Restmenge an Verbindung (V) wurde unter Zusatz von 0.1-0.5% Essigsäure zum Laufmittel eluiert. Nach Einengen der Fraktionen und mehrstündigem Trocknen der Substanz bei 0.1 Torr wurden 6,85 g (79,3% der Theorie) der Substanz (IV) als hochviskoses Öl erhalten. Die so erhaltene Substanz ist nach HPLC und 1H-NMR identisch mit der in DOS 2845770 beschriebenen Substanz.

## Patentansprüche

1. Verfahren zur Herstellung von Carbacyclinen der Formel I

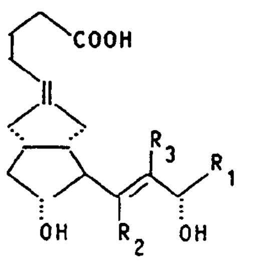

(I),

worin

$R_1$ eine geradkettige oder verzweigte $C_2$-$C_7$-Alkinyl-Gruppe und

$R_2$, $R_3$ bedeuten können,

dadurch gekennzeichnet, daß man ein bicyclisches Keton der Formel II

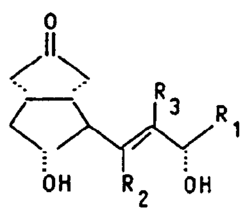

(II),

worin $R_1$, $R_2$ und $R_3$ die oben angegebenen Bedeutungen haben, mit 5-10 Equivalenten eines Reagenz der Formel VI,

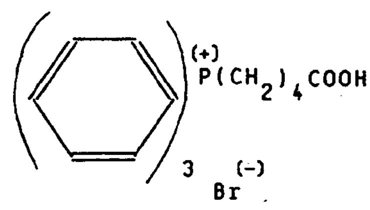

(VI),

in Gegenwart von 8-12 Equivalenten einer Base, bei 0-40°C in 0,5 bis 4 Std. in den Lösungsmitteln Dimethoxyethan, 1,3-Dimethylimidazolidin-2-on, Dimethylformamid, Dimethylsulfoxid, Tetrahydrofuran, Toluol, Dibenzo-18-krone-6 oder in deren Gemischen umsetzt.

## Claims

1. A process for the preparation of carbacyclines of the formula I

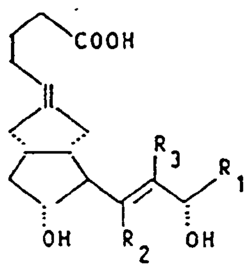

(I)

wherein
$R_1$ may be a straight-chain or branched $C_2$-$C_7$alkynyl group, and
$R_2$, $R_3$ may be hydrogen,
characterised in that a bicyclic ketone of the formula II

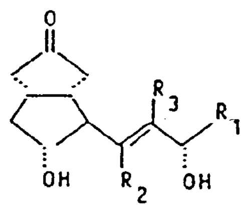

(II),

wherein $R_1$, $R_2$ and $R_3$ have the meanings given above, is reacted with from 5 to 10 equivalents of a reagent of the formula VI

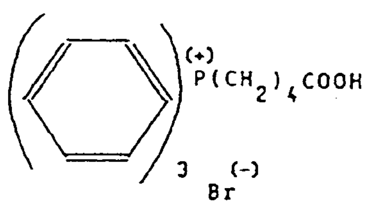

(VI)

in the presence of from 8 to 12 equivalents of a base, at from 0 to 40°C in the course of from 0.5 to 4 hours in the solvents dimethoxyethane, 1,3-dimethylimidazolidin-2-one, dimethylformamide, dimethyl sulphoxide, tetrahydrofuran, toluene, dibenzo-18-crown-6 or in mixtures thereof.

## Revendications

1. Procédé pour préparer des carbacyclines répondant à la formule I :

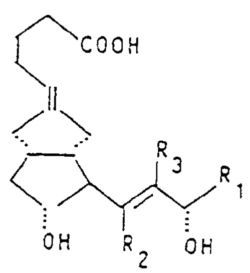

(I)

dans laquelle :

$R_1$ représente un radical alcynyle en $C_2$-$C_7$ linéaire ou ramifié et
$R_2$ et $R_3$ représentent chacun l'hydrogène,
procédé caractérisé en ce qu'on fait réagir une cétone bicyclique répondant à la formule II :

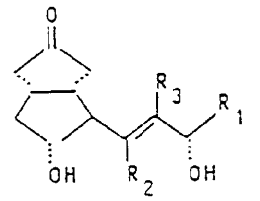

(II)

dans laquelle $R_1$, $R_2$ et $R_3$ ont les significations qui leur ont été données ci-dessus, avec de 5 à 10 équivalents d'un réactant de formule VI .

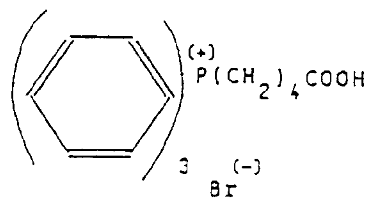

(VI),

en présence de 8 à 12 équivalents d'une base, à une température de 0 à 40°C, pendant 0,5 à 4 heures, dans un solvant pris dans l'ensemble constitué par le diméthoxyéthane, la 1,3-diméthyl-imidazolidine-2-one, le diméthylformamide, le diméthylsulfoxyde, le tétrahydrofuranne, le toluène et la dibenzo-18-couronne-6 ou dans des mélanges de ces solvants.